Europäisches Patentamt

European Patent Office

Office européen des brevets

(11) Publication number : **0 465 037 A1**

# (12) EUROPEAN PATENT APPLICATION

(21) Application number : 91305400.3

(22) Date of filing : 14.06.91

(51) Int. Cl.⁵ : **G03G 11/00, C11D 7/50**

(30) Priority : **29.06.90 US 545861**

(43) Date of publication of application :
**08.01.92 Bulletin 92/02**

(84) Designated Contracting States :
**DE FR GB IT**

(71) Applicant : **MINNESOTA MINING AND MANUFACTURING COMPANY**
**3M Center, P.O. Box 33427**
**St. Paul, Minnesota 55133-3427 (US)**

(72) Inventor : **Audenaert, Frans, c/o Minnesota Mining and**
**Manufact. Co., 2501 Hudson Road, P.O. Box 33427**
**St. Paul, Minnesota 55133-3427 (US)**
Inventor : **DeWinter, Constant F. c/o Minnesota Mining and**
**Manufact. Co., 2501 Hudson Road, P.O. Box 33427**
**St. Paul, Minnesota 55133-3427 (US)**
Inventor : **Flynn, Richard M. c/o Minnesota Mining and**
**Manufact. Co., 2501 Hudson Road, P.O. Box 33427**
**St. Paul, Minnesota 55133-3427 (US)**

(74) Representative : **Baillie, Iain Cameron et al**
**c/o Ladas & Parry Altheimer Eck 2**
**W-8000 München 2 (DE)**

(54) Solvent composition.

(57) Homogeneous, organic, liquid solvent compositions are described which include (a) a fluorine-free organic liquid, (b) a perfluorinated organic liquid, and (c) a co-solvent which is miscible with components (a) and (b). The compositions have utility in diverse applications, such as cleaning and degreasing, aerosol compositions, fixing of toner powder, heat transfer media, thermal and electrical insulation, etc. The solvent compositions have low ozone depletion potentials. This makes them attractive as replacement solvents for conventional chlorofluorocarbonss.

EP 0 465 037 A1

This invention relates to solvent compositions. More particularly, this invention relates to solvent compositions which include perfluorinated organic liquid. In another aspect this invention relates to solvent compositions having utility in diverse applications such as fixing of toner powder, cleaning and degreasing, de-watering, thermal insulation, heat transfer media, electrical insulation, aerosol compositions, etc.

Recently there has arisen a concern over the potential of certain chlorine containing compounds (the chlorofluorocarbons or CFCs) to cause significant depletion of the Earth's protective ozone layer in the upper stratosphere. The ozone layer functions as a protective layer by absorption of some of the harmful ultraviolet radiation from the sun. Thus, depletion of the ozone layer is expected to be directly correlated with an increase in human skin cancer as well as causing other detrimental environmental effects. An article in Chemical and Engineering News, Oct. 9, 1989, page 4, "CFC Substitutes...", states: "The urgent need to find CFC alternatives is underscored by the latest news from Antarctica: The 'ozone hole' has returned with a vengeance. Atmospheric scientists have determined that chlorine from CFCs is the primary factor in the seasonal loss of stratospheric ozone that has been recurring each Antarctic spring for a decade."

The Montreal Protocol to which the United States is signatory has been written to control the compounds which cause the most significant depletion of the ozone layer. Certain compounds having high ozone depletion potentials will begin to be restricted in 1994 and may be banned altogether by the year 2000.

Thus, there is an urgent need to replace CFCs in industrial applications. This includes use of such compounds in solvents, aerosols, refrigerants, etc. Such compounds are also used in the fixing of toner powders, e.g., in laser printers.

One of the solvents used in fixing of toner powders is the CFC solvent, 1,1,2-trichlorotrifluoro-ethane, or CFC-113, which is used, for example, as a mixture with dichloromethane or with acetone, as described in United States Patent No. 4,311,723 (Mugrauer). This solvent has an ozone depletion potential of 0.8, a high value compared with the hydrochlorofluorocarbons (HCFCs) which are being used and proposed as replacement solvents. Some of the HCFC candidates and their ozone depletion potential (in parentheses) include: HCFC-134a, $CH_2FCF_3$ (0); HCFC-123, $CHCl_2CF_3$ (0.016); HCFC-141b, $CH_3CCl_2F$ (0.081); and HCFC-22, $CHClF_2$ (0.053).

United States Patent No. 4,828,751 (Kremer) discloses solvents for the cleaning of silicon wafers consisting essentially of about 30 parts to about 90 parts of component (a) a haloalkylhydrocarbon, e.g., 1,1,2-trichlorotrifluoroethane, perfluorohexane, etc., and about 10 to about 70 parts of component (b) of a partially fluorinated alcohol, e.g., trifluoroethanol, 2,2,3,3,3-pentafluoropropanol, etc. Minor amounts, less than 5 parts/100 parts by weight, preferably less than 0.1 part/100 parts, of other volatile ingredients may also be present, e.g., alcohols, ethers, ketones and esters.

The above cited United States Patent No. 4,311,723 (Mugrauer) discloses and claims a method for vapor-fixing a toner on a data-carrier comprising contacting such toner with a solvent composed of an azeotropic mixture consisting of about 50.5 weight % 1,1,2-trichlorotrifluoroethane and about 49.5 weight % dichloromethane or an azeotropic mixture consisting of a mixture of 87.5 weight %, 1,1,2-trichlorotrifluoroethane and about 12.5 weight % acetone.

One of the objects of the invention is to provide solvent compositions having lower ozone depletion potentials than the conventional compositions containing chlorofluorocarbons.

Another object of the invention is to provide suitable solvent compositions capable of fixing toner powder images on a substrate and which have lower ozone depletion potentials than compositions containing chlorofluorocarbons.

Other objects of the invention are to provide solvent compositions useful for rinsing or cleaning electronic printed circuit boards, and to provide other compositions useful in aerosol compositions, for example.

Other objects of this invention will be apparent from the detailed description hereinafter.

In one embodiment of the invention there are provided homogeneous organic liquid solvent compositions which have lower ozone depletion potentials than conventional compositions containing chlorofluorocarbons. In one embodiment of the invention the solvent compositions comprise:

A. fluorine-free organic liquid selected from the group consisting of ketones, alcohols, esters, nitriles, hydrocarbons, ethers, amides, and chlorinated compounds,

B. a perfluorinated organic liquid, and

C. a compatibilizing co-solvent which is miscible with components A and B.

In another embodiment of the invention the solvent composition is used to fix or fuse a toner powder on a data-carrying substrate. The toner powder is contacted with hot vapors of the solvent composition.

The solvent compositions described herein are very useful in a variety of applications. For example, they may be used for fixing toner powders, or as refrigerants, as heat transfer media, insulation, cleaning and degreasing compositions, expansion agents for foam, solvents for organic compounds, especially fluorochemical compounds and fluorinated polymers, etc. The solvent composition is also useful as a water displacement solvent for the spot-free drying of mechanical or electronic parts.

The solvent compositions are especially useful in aerosol compositions. Present CFC alternatives are either very flammable (e.g., dimethyl ether, hydrocarbons) or toxic (1,1,1-trichloroethane, or other chlorinated solvents). Perfluorinated liquids cannot be used alone as solvents in aerosol compositions mainly because of their poor solvent properties for organic products.

The solvent compositions of the invention exhibit low or zero ozone depletion potential. They also are nonflammable (or of low flammability), and have low toxicity.

The liquid solvent compositions of this invention are homogeneous solutions at room temperature (about 25°C.). Since organic liquid compounds, especially polar compounds, are relatively insoluble in perfluorinated liquids, it is surprising that they can be made into a homogeneous solution by means of a third solvent.

The fluorine-free organic liquids (Component A) which are useful in this invention can be chlorinated and can contain hetero atoms selected from oxygen, nitrogen, and sulfur atoms. Preferably such liquids have a boiling point in the range of about 25 tc 130°C. (even more preferably, about 30 to 100°C.). Such liquids are selected from ketones, alcohols, esters, nitriles, hydrocarbons, ethers, amides and chlorinated compounds. The preferred organic liquids are polar. "Polar" as it is used herein means compounds having dipoles.

Examples of useful ketones include acetone, methyl ethyl ketone, methyl isobutyl ketone, 2-butanone, 4-methyl-2-pentanone, 5-methyl-2-hexanone, etc. Examples of useful esters include ethyl acetate, propyl acetate, propylene glycol monomethylether acetate, 2-ethoxyethanol acetate, 2-butoxyethanol acetate, etc. Examples of useful alcohols include ethanol, propanol, isopropanol, butanol, 2-ethyoxyethanol, 2-butoxyethanol, propylene glycol, propylene glycol monomethylether, etc.

Examples of useful nitriles include acetonitrile, propionitrile, etc. Examples of useful amides include dimethylformamide, dimethylacetamide, N-methylpyrrolidone, etc.

Examples of useful hydrocarbons include hexane, octane, toluene, etc. Examples of useful ethers include diethylether, ethylene glycol dimethyl ether, tetrahydrofuran, etc.

Examples of useful chlorinated hydrocarbons include 1,1,1-trichloroethane, methylene chloride, chloroform, perchloroethylene, etc.

Mixtures of the foregoing types of organic liquids may also be used. Further, useful organic liquids include compounds which have more than one type of polar moiety, e.g., 2-chloroethanol and 2-hydroxyethyl acetate.

When the solvent composition is to be used for fixing or fusing toner powder, a particularly preferred polar liquid component comprises acetone. Preferably the acetone is present in an amount of about 5 to 15% by weight (and more preferably about 8 to 12% by weight).

The resulting solvent composition can be used to fix or fuse toner powder images on a substrate to give an appearance identical to that obtained using the conventional CFC-113/acetone binary solvent system.

Perfluorinated liquids (component B) of this invention, include those perfluorinated compounds boiling in the range of about 25°C. to about 130°C., and include perfluoroalkanes, and perfluorinated compounds containing one or more heteroatoms, e.g., oxygen and nitrogen atoms, such as perfluoroethers, perfluoroamines and the like. The term "perfluorinated" as used herein includes organic compounds in which all (or essentially all) of the hydrogen atoms are replaced with fluorine atoms.

Specific useful perfluorinated liquids include the following compounds:

## TABLE I

| Compound (Component B) | Boiling point, °C. |
|---|---|
| 1. perfluoropentane, $C_5F_{12}$ | 30 |
| 2. 1,2-bis(trifluoromethyl)hexafluoro-cyclobutane, $1,2-(CF_3)_2-c-C_4F_6$ | 46 |
| 3. perfluoro-4-methylmorpholine, $CF_3N$⟨ F ⟩O | 51 |
| 4. perfluoro-2-ethyltetrahydrofuran, $CF_2$——$CFC_2F_5$ / $CF_2$ $CF_2$ / O | 56 |
| 5. perfluorohexane, $C_6F_{14}$ | 56 |
| 6. perfluorotriethylamine, $(C_2F_5)_3N$ | 71 |
| 7. perfluoroheptane, $C_7F_{16}$ | 84 |
| 8. perfluorodibutylether, $C_4F_9OC_4F_9$ | 102 |

and mixtures of these and other perfluorinated liquids.

Preferred perfluorinated liquids include the perfluoroalkanes 1, 3, 5 and 7 from the above table. These materials are readily available and allow one to prepare useful solvent compositions boiling in the range of about 25°C. to about 130°C.

Since perfluorinated liquids do not have a detrimental effect on the ozone layer and hence are exempt from the restrictions of the Montreal Protocol, the ozone depletion potential of the resulting compositions will ultimately depend on the identity of the third solvent (Component C) which is the co-solvent for the perfluorinated liquid B as well as a solvent for the organic liquid solvent A. If this third solvent and solvent A do not contain chlorine (or bromine) the ozone depletion potential of the resulting blend will be zero. Many of the compositions of this invention thus have an ozone depletion potential of zero.

It has been found that certain of the HCFCs, some of which are described above, also serve as the third solvent, component C, in this invention. In this case, the ozone depletion potential of the resulting mixture can be calculated in a straightforward manner. The most advantageous compositions of this class will however have extremely low ozone depletion potentials and hence will not likely be regulated in the foreseeable future.

The solvents which have been shown to be useful as the third solvent, component C of this invention, include the following compounds:

1,3-bis(trifluoromethyl)benzene, $1,3-C_6H_4(CF_3)_2$
1-hydropentadecafluoroheptane, $C_7F_{15}H$
2,2,3,3,4,4,4-heptafluorobutanol, $CF_3CF_2CF_2CH_2OH$
2,2,3,3,3-pentafluoropropanol, $CF_3CF_2H_2OH$
methyl heptafluorobutyrate, $C_3F_7COOCH_3$
hexafluoro-2-propanol, $(CF_3)_2CHOH$
and the like, and mixture of these and related compounds.

In addition to the above-mentioned solvents which all afford a final composition of zero ozone depletion potential, there are some solvents containing hydrogen from the class of HCFCs which are also effective as the third solvent C. These include the following compounds:

1,1-dichloro-2,2,2-trifluoroethane, $CF_3CHCl_2$ (HCFC-123)
1-chloro-3,3,3-trifluoropropane, $CF_3CH_2CH_2Cl$ (HCFC-253)
1,2-dichlorotrifluoroethane, $CF_2ClCHFCl$ (HCFC-123a)
1,1-dichloro-1-fluoroethane, $CH_3CFCl_2$ (HCFC-141b)
1,2-dichloro-1,1-difluoroethane, $CF_2ClCH_2Cl$ (HCFC-132b)
1,2,2-trichloro-1,1-difluoroethane, $CF_2ClCHCl_2$ (HCFC-122)
and mixtures of these and similar materials.

Of the HCFC compounds, HCFC-123 is especially preferred as component C. If HCFC compounds are used as the co-solvent C, then a perfluorinated solvent B may not be required.

The co-solvent C should not exceed about 50% by weight of the solvent composition. Preferably, the co-solvent does not exceed about 30% by weight of the composition.

The amount of co-solvent C present in the solvent composition can be determined empirically. The amount of co-solvent C required to effect a solution of components A and B will vary, depending upon the nature of all of the components. Some solvent combinations may not be suitable for certain applications because of flammability.

The most preferred composition for lowest flammability uses HCFC-123 as the third solvent. CFC-113 itself can also be used as the third solvent but requires a relatively larger amount to solubilize an equivalent amount of acetone in perfluorohexane and hence would only offer a relatively small improvement in the ozone depletion potential of the mixture compared with the compositions of this invention. For example for 7.1 wt.% acetone, 28.6 wt.% CFC-113 is required and the ozone depletion potential of this mixture is then 0.23, which is still a significant improvement over the cited example in U.S. Patent 4,311,723 in which the ozone depletion potential is 0.70.

In addition to acetone as the toner curing or fusing component A of the solvent compositions of this invention, other ketones such as 2-butanone, as well as other classes of solvents can be used to cure the toner. These include esters, e.g., ethyl acetate, alcohols, e.g., ethanol, or chlorinated compounds such as methylene chloride. These compounds can also be solubilized in perfluorinated liquids in a manner similar to acetone as described above. However, even though methylene chloride could be used as component A as indicated, and made fully soluble by the proper choice of a third solvent C, its toxicity prevents its use for this type of application in a practical sense.

Because acetone is miscible with many HCFCs, compositions containing acetone and HCFC can be used to cure or fix toner powder images in an acceptable manner. Of these HCFCs which can be used in this way without the use of a perfluorinated co-solvent, HCFC-123 is especially preferred. An 11% by weight solution of acetone in HCFC-123 has an ozone depletion potential of 0.014.

For fixing toner powder images, it has been found that compositions comprising 5 to 15% by weight of component A and 85 to 95% by weight of component C are quite useful.

Acetone is miscible with certain hydrogen-containing perfluorocarbon compounds such as $C_7F_{15}H$. Such mixtures may also be useful as solvent compositions of this invention, and furthermore they have an ozone depletion potential of zero.

Toners which can be fixed or cured using the solvent compositions of this invention are colored or pigmented, (e.g., with carbon black) thermoplastic resin-based, free-flowing, finely-divided particles. See for example, Kirk-Othmer Encyclopedia of Chemical Technology 3rd Ed. Vol. 8, page 794-826 "Electrophotography", John Wiley and Sons, New York, 1979.

The liquid solvent compositions of this invention are further illustrated by means of the following non-limiting examples.

## Example 1

This example will demonstrate that various ratios or proportions of the same three solvents can be found which will afford a homogeneous solution. For the perfluorohexane/acetone/1,3-bis(trifluoromethyl)benzene (B/A/C) system, the following combinations all provide a homogeneous solution at 25°C. All percentages given are in weight percent and in the order above.

| Run no. | Component: | Weight percent of components | | |
|---|---|---|---|---|
| | Compound: | B $C_6F_{14}$ | A $CH_3COCH_3$ | C $1,3-C_6H_4(CF_3)_2$ |
| 1. | | 98.0 | 1.0 | 1.0 |
| 2. | | 91.3 | 2.8 | 5.8 |
| 3. | | 82.6 | 4.3 | 13.0 |
| 4. | | 73.3 | 7.9 | 18.8 |
| 5. | | 68.3 | 9.8 | 21.9 |
| 6. | | 64.6 | 11.4 | 23.9 |
| 7. | | 73.3(a) | 7.6 | 19.1 |

(a) Perfluoromethylcyclohexane used in place of $C_6F_{14}$.

Example 2

These are examples of other compositions in which the perfluorohexane B is held constant but the third co-solvent C is varied. All percentages are in weight percent. The compositions are prepared at room temperature and are homogeneous.

| Run no. | Component: | Weight percent of components | | |
|---------|-----------|------|------|------|
| | | B | A | C |
| | Compound: | $C_6F_{14}$ | $CH_3COCH_3$ | (See footnotes) |
| 1. | | 66.2 | 7.4 | 26.5 (a) |
| 2. | | 73.2 | 8.1 | 18.7 (b) |
| 3. | | 75.0 | 8.3 | 16.5 (c) |
| 4. | | 75.6 | 8.4 | 16.0 (d) |
| 5. | | 71.0 | 8.0 | 21.0 (d) |
| 6. | | 47.0 | 5.0 | 48.0 (e) |
| 7. | | 74.0 | 8.0 | 18.0 (f) |
| 8. | | 76.0 | 9.0 | 15.0 (g) |
| 9. | | 77.0 | 9.0 | 14.0 (h) |
| 10. | | 66.6 | 9.1 | 24.3 (i) |

(a) 1-hydropentadecafluoroheptane, $C_7F_{15}H$

(b) methyl heptafluorobutyrate, $C_3F_7COOCH_3$

(c) 2,2,3,3,4,4,4-heptafluorobutanol $C_3F_7CH_2OH$

(d) 1,1,1,3,3,3-hexafluoropropanol, $(CF_3)_2CHOH$

(e) benzotrifluoride, $C_6H_5CF_3$

(f) undecafluorocyclohexyl methanol, $c-C_6F_{11}CH_2OH$

(g) 1,1,2.2-tetrahydroperfluorooctanol, $C_6F_{13}CH_2CH_2OH$

(h) 1,1-dihydroperfluorooctanol, $C_7F_{15}CH_2OH$

(i) 2,2,3,3,3-pentafluoropropanol, $C_2F_5CH_2OH$

Example 3

These compositions are examples of further combinations or blends which can be used to solubilize acetone in perfluorohexane in which the third solvent C is of the class of HCFCs. Run no. 1 utilizes CFC-113 in a comparative example.

| | | Weight percent Of components | | |
|---|---|---|---|---|
| Run no. | Component: | B | A | C |
| | Compound: | $C_6F_{14}$ | $CH_3COCH_3$ | (see footnotes) |
| 1. | | 64.3 | 7.1 | 28.6 (a) |
| 2. | | 67.1 | 10.9 | 22.0 (b) |
| 3. | | 62.4 | 6.9 | 33.8 (c) |
| 4. | | 72.9 | 8.1 | 19.0 (d) |
| 5. | | 63.5 | 7.1 | 29.4 (e) |
| 6. | | 59.6 | 6.6 | 33.8 (f) |
| 7. | | 60.0 | 6.7 | 33.3 (g) |
| 8. | | 64.7 | 7.2 | 28.0 (h) |

a. $CF_2ClCFCl_2$ (CFC-113)

b. $CF_3CHCl_2$ (HCFC-123)

c. $CF_3CH_2CH_2Cl$ (HCFC-253)

d. $CF_2ClCFHCl$ (HCFC-123a)

e. $CH_3CFCl_2$ (HCFC-141b)

f. $CF_2ClCH_2Cl$ (HCFC-132b)

g. $CF_2ClCHCl_2$ (HCFC-122) -- homogeneous only at temperatures greater than 33°C.

h. 80/20 wt. % mixture of HCFC-141b/HCFC-123 (Genesolv$^{TM}$ 2020 available from Allied-Signal)

Example 4

These compositions show that a combination of perfluorinated liquids can be used instead of one single liquid. For these examples the perfluorinated liquids used were perfluoropentane and perfluorohexane and were used as received.

| | | Weight percent of components | | |
|---|---|---|---|---|
| Run no. | Component: | B | A | C |
| | Compound: | $C_6F_{14}/C_5F_{12}$ | $CH_3COCH_3$ | (see footnotes) |
| 1. | | 38.5/30.8 | 7.7 | 23.1 (a) |
| 2. | | 37.0/17.0 | 10.0 | 36.0 (b) |

a. 1-hydropentadecafluoroheptane, $C_7F_{15}H$

b. 1,3-bis(trifluoromethyl)benzene, $1,3-C_6H_4(CF_3)_2$

Example 5

In order to demonstrate that a particular composition was capable of curing the toner used in the fixing device, the following experiment was carried out. The toner used was "Toner 22 for Laser Printer, S26312-F91, Colour: Black"; available from Siemens AG. The composition of Example 1, Run no. 4, was placed in a 500 mL. open beaker to a depth of about one centimeter. The beaker was placed on a stirred hot plate and heated to gentle reflux. A sample of paper onto which the toner had been loosely spread with a spatula was very briefly (less than one second) immersed completely into the vapor zone. Upon removal of the paper it was observed that the toner had now become fixed to the paper and could no longer be removed by simple mechanical abrasion. Prior to cure, the toner was easily and completely removed. Testing of the toner fixing effectiveness of other compositions of this inventions showed that those compositions containing at least about 7.0 wt. % acetone were also effective using the above test.

Example 6

These compositions illustrate that compounds other than acetone can also be solubilized in perfluorinated solvents by the use of a suitable ternary solvent.

| | | Weight percent of components | | |
|---|---|---|---|---|
| Run no. | Component: | B | A | C |
| | Compound: | $C_6F_{14}$ | (see footnotes) | $CF_3CHCl_2$ |
| 1. | | 67.5 | 11.9 (a) | 20.6 |
| 2. | | 69.7 | 12.3 (b) | 18.0 |
| 3. | | 56.7 | 10.0 (c) | 33.3 |
| 4. | | 63.9 | 11.3 (d) | 24.8 |
| 5. | | 72.0 | 12.7 (e) | 15.3 |
| 6. | | 56.7 | 10.0 (f) | 33.3 |
| 7. | | 69.7 | 12.3 (g) | 18.0 |
| 8. | | 77.3 | 13.6 (h) | 9.1 |
| 9. | | 65.4 | 11.5 (i) | 23.1 |
| 10. | | 56.6 | 11.3 (c) | 32.0 (j) |
| 11. | | 81.1 | 9.0 (k) | 9.9 |

a. 2-butanone, $CH_3COC_2H_5$

b. 4-methyl-2-pentanone, $CH_3COCH_2CH(CH_3)_2$

c. ethanol, $C_2H_5OH$

d. 2-propanol, $CH_3CH(OH)CH_3$

e. 2-methyl-2-propanol, $(CH_3)_3COH$

f. 2-methyl-1-propanol, $CH_3CH(CH_3)CH_2OH$

g. dichloromethane, $CH_2Cl_2$

h. chloroform, $CHCl_3$

i. toluene, $C_6H_5CH_3$

j. 2,2,3,3,3-pentafluoropropanol, $C_2F_5CH_2OH$

k. ethyl acetate, $CH_3CO_2C_2H_5$

Example 7

Similar compositions are prepared using the procedures and components of Example 6, except that $CF_3CHCl_2$ has been replaced with 1,3-bis(trifluoromethyl)benzene as the ternary solvent. The compositions prepared are shown in the following table.

| Run no. | Component: | Weight percent of components | | |
|---------|-----------|:---:|:---:|:---:|
| | | B | A | C |
| | Compound: | $C_6F_{14}$ | (*See below for entries) | $1,3-C_6H_4(CF_3)_2$ |
| 1. | | 65.4 | 11.5 (a) | 23.1 |
| 2. | | 68.5 | 12.1 (b) | 19.4 |
| 3. | | 54.5 | 9.6 (c) | 35.9 |
| 4. | | 61.6 | 10.9 (d) | 27.5 |
| 5. | | 68.5 | 12.1 (e) | 19.4 |
| 6. | | 53.1 | 9.4 (f) | 37.5 |
| 7. | | 69.7 | 12.3 (g) | 18.0 |
| 8. | | 72.0 | 12.7 (h) | 15.2 |
| 9. | | 65.4 | 11.5 (i) | 23.1 |
| 10. | | 79.0 | 9.0 (k) | 12.0 |
| 11. | | 58.0 | 6.0 (l) | 36.0 |
| 12. | | 88.0 | 10.0 (m) | 2.0 |

*For runs 1-10 see footnotes of Example 6.

For runs 11 and 12, (l) dimethylformamide, $CH_3CON(CH_3)_2$; (m) 1,1,1-trichloroethane, $CH_3CCl_3$.

Example 8

Cleaning of solder flux (611F from Alpha Metals) from printed circuit boards after repair in the field is presently done with CFC-113 using a spray can.

A replacement homogeneous solvent system with zero ozone depletion potential was prepared by placing a mixture of 56 g of perfluorohexane and 4 g of ethanol in a 250 mL bottle and adding candidate co-solvents in 1 g increments and shaking the bottle between additions to check for homogeneity. The following candidate co-solvents did not give a homogeneous solution in these studies: hexane, heptane, perchloroethylene, trichloroethylene and chloroform.

A satisfactory co-solvent was found to be 1,3-bis(trifluoromethyl)benzene. The homogeneous, ternary solvent system had the following composition (percent by wt.)

66% perfluorohexane (B solvent)

5% ethanol (A solvent)

29% 1,3-bis(trifluoromethyl)benzene (C solvent) This solvent system was effective in cleaning flux from a printed circuit board.

Example 9

A homogeneous solution of 1 wt. % fluorochemical oil and water repellant composition for the spray (aerosol, etc.) treatment of textiles and leather to impart soiling resistance thereto was prepared from 2.5 g of FX-3532 (a 40 wt. % solution of fluorochemical polymer in ethyl acetate, available from 3M), 40.0 g of 1,3-bis(trifluoromethyl)benzene and 57.5 g perfluorohexane. The composition of the ternary solvent system described in this example was as follows:

58.1 % perfluorohexane (B solvent)

1.5 % ethyl acetate (A solvent)

40.4 %    1,3-bis(trifluoromethyl)benzene (C solvent)

**Claims**

1. A homogeneous, organic, liquid solvent composition comprising:
   (a) a fluorine-free organic liquid;
   (b) a perfluorinated organic liquid, and
   (c) a co-solvent which is miscible with components (a) and (b).

2. A composition according to claim 1, wherein component (a) is selected from the group consisting of ketones, alcohols, esters, and chlorinated compounds.

3. A composition according to any of claims 1 to 2, wherein component (a) is selected from the group consisting of ketones, alcohols, esters, nitriles, amides, hydrocarbons, ethers, and chlorinated compounds, and wherein component (b) has a boiling point in the range of about 25°C. to 100°C.

4. A composition according to any of claims 1 to 3, wherein component (a) is present in an amount of about 5 to 15% by weight; component (b) is present in an amount of about 35 to 85% by weight; and component (c) is present in an amount of about 10 to 50% by weight.

5. A composition according to any of claims 1 to 4, wherein component (b) comprises a perfluoroalkane.

6. A composition according to any of claims 1 to 5, wherein component (c) is selected from the group consisting of 1,3-bis(trifluoromethyl) benzene;
   1-hydropentadecafluoroheptane;
   2,2,3,3,4,4,4-heptafluorobutanol; methyl heptafluorobutyrate; hexafluoro-2-propanol;
   1,1-dichloro-2,2,2-trifluoroethane;
   1-chloro-3,3,3-trifluoropropane;
   1,2-dichlorotrifluoroethane; 1,1-dichloro-1-fluoroethane;
   1,2-dichloro-1,1-difluoroethane;
   1,2,2-trichloro-1,1-difluoroethane;
   2,2,3,3,3-pentafluoropropanol.

7. A method for fusing a toner powder on a substrate by contacting said toner powder with a liquid composition, characterized in that the liquid composition comprises the homogeneous, organic, liquid solvent composition according to any of claims 1 to 6.

8. A method for cleaning flux from a printed circuit board by contacting said flux with a liquid composition, characterized in that the liquid composition comprises the homogeneous, organic, liquid solvent composition according to any of claims 1 to 6.

9. A method for treating a surface with a fluorochemical compound in an aerosol composition to impart oil and water repellency to said surface, characterized in that the aerosol composition comprises the homogeneous, organic, liquid solvent composition according to any of claims 1 to 6.

| | European Patent Office | EUROPEAN SEARCH REPORT | Application Number |
| --- | --- | --- | --- |

EP 91 30 5400

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| X | US-A-3 929 662 (R. M. G. BOUCHER)<br>* abstract; claims 1-7 *<br>--- | 1,8 | G03G11/00<br>C11D7/50 |
| X | DATABASE WPIL, no. 80-74980C<br>Derwent Publications Ltd., London, GB,<br>& SU-A-720008 ( Instr. Mfr. Res. Techn.)<br>* the whole abstract *<br>--- | 1,2,4,8 | |
| D,A | GB-A-2 029 325 (SIEMENS AG)<br>* claims 1-7 *<br>--- | 1-9 | |
| A | GB-A-2 188 059 (KALI-CHEMIE AG)<br>* claims 1-18 *<br>--- | 1-9 | |
| A | GB-A-1 544 841 (PLESSEY CO., LTD.)<br>* claims 1-6 *<br>----- | 1-9 | |
| | | | TECHNICAL FIELDS SEARCHED (Int. Cl.5)<br><br>G03G<br>C11D |

The present search report has been drawn up for all claims

| Place of search | Date of completion of the search | Examiner |
| --- | --- | --- |
| THE HAGUE | 08 OCTOBER 1991 | HINDIAS E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)